# EUROPEAN PATENT SPECIFICATION

(11) **EP 2 499 957 B1**
(45) Date of publication and mention of the grant of the patent: **09.10.2013**
(21) Application number: 12157086.5
(22) Date of filing: 27.02.2012
(51) Int. Cl.: A61B 1/00

(54) **Self-propellable apparatus**
Selbstantreibende Vorrichtung
Appareil automoteur

(30) Priority: 15.03.2011 JP 2011056346
(43) Date of publication of application: 19.09.2012
(73) Proprietor: Fujifilm Corporation, Minato-ku Tokyo (JP)
(72) Inventor: Ohta, Yasunori, Ashigarakami-gun, Kanagawa (JP); Ashida, Tsuyoshi, Ashigarakami-gun, Kanagawa (JP); Nakamura, Takayuki, Ashigarakami-gun, Kanagawa (JP); Yamakawa, Shinichi, Ashigarakami-gun, Kanagawa (JP); Iwasaka, Masayuki, Ashigarakami-gun, Kanagawa (JP)
(74) Representative: Klunker . Schmitt-Nilson . Hirsch

(56) References cited:
- US-A1- 2005 154 278

## Description

### BACKGROUND OF THE INVENTION

### 1. Field of the Invention

The present invention relates to a self-propellable apparatus according to the preamble of claim 1. The apparatus is used with being attached to an insert section of an endoscope.

### 2. Description Related to the Prior Art

An endoscope is widely known as a medical instrument that is inserted into a patient' s body to obtain a view of the interior of the body for treatment and diagnosis. The endoscope is provided with an insert section to be introduced into the patient' s body and a handling section for manipulating the insert section. The insert section is bendable at its front end portion by the manipulation from the handling section, so as to navigate through a body cavity, body canal, or body lumen having curves and turns. However, the insert section is difficult by the manipulation to introduce into the unfixed and free body lumen including the sigmoid colon and transverse colon. The introduction sometimes causes discomfort and pain to the patient, depending on skill of the manipulation by an endoscopist.

There is known a self-propellable apparatus that helps the introduction of the insert section, as described in US Patent No. 7,736,300 corresponding to Japanese Unexamined Patent Application Publication (Translation of PCT Application) No. 2009-513250. In this apparatus, a hollow and toroidal bladder is attached to the front end portion of the insert section. Circulating the bladder propels the insert section into the depths of the body lumen, for example, the intestine.

In the apparatus of the above US Patent No. 7,736,300, however, a mechanism that holds and circulates the bladder is necessarily provided inside the bladder, and causes complex structure and high cost. Also, since the insert section is propelled by use of the friction between the bladder and an interior wall of the body lumen, enough propulsive force cannot be obtained when the outside diameter of the bladder is small. On the other hand, the large outside diameter of the bladder increases a physical burden on the patient.

In accordance with the preamble of claim 1, US 2005/154278 A1 discloses a self-propellable apparatus including a set of angularly displaced tracking members held by a carrier. Each tracking member includes a belt consisting of an endless web fed around a pair of pulleys. By driving the pulleys, the outer surface of the traction members which contact an interior wall of a body lumen propels the apparatus.

### SUMMARY OF THE INVENTION

An object of the present invention is to provide at low manufacturing cost a self-propellable apparatus that can produce sufficient propulsive force with alleviating a burden on a patient.

To achieve the above and other objects of the present invention, a self-propellable apparatus according to the present invention includes a feeding roller and a belt drawing-out mechanism. On the feeding roller, one end of a belt is wound. The belt drawing-out mechanism is disposed behind the feeding roller. The belt drawing-out mechanism draws out the belt from the feeding roller to produce a propulsive force to the front end portion, when said belt is pressed against an interior wall of the body lumen.

Also, the self-propellable apparatus preferably includes a balloon disposed on an inner side of the belt. The balloon presses the belt against an interior wall of a body lumen. The balloon is changeable between an inflated state and a deflated state.

A plurality of sets each of which includes the feeding roller and the belt drawing-out mechanism may be provided around a periphery of the insert section. Alternatively, a plurality of sets each of which includes the feeding roller, the belt drawing-out mechanism, and the balloon may be provided around the periphery of the insert section.

The belt drawing-out mechanism may include a worm wheel rotated by the outside power source to move the belt. The belt drawing-out mechanism may further include a driven roller disposed behind the worm wheel. The driven roller turns the belt drawn out from the feeding roller to the worm wheel and presses the belt against the worm wheel.

The self-propellable apparatus may further include a fixed cylinder, a gear barrel, and a belt holder. The fixed cylinder is fitted onto the front end portion of the insert section. The gear barrel is rotatably fitted on the fixed cylinder. The worm wheel is disposed on an outer periphery of the gear barrel. The belt holder is held by the fixed cylinder. The belt holder has the feeding roller, the worm wheel, and the driven roller attached thereto.

The belt drawing-out mechanism may further include a winding roller to which the other end of the belt is secured. The winding roller is disposed in an opposite side of the driven roller with respect to the worm wheel, and rotated in conjunction with the worm wheel to wind up the belt fed from the worm wheel. The winding roller preferably steps back against a bias of a spring by a distance corresponding to a winding diameter of the belt.

In another case, the belt drawing-out mechanism may further include space formed between the fixed cylinder and the belt holder. The space holds the belt fed from the worm wheel. The belt drawing-out mechanism may further include a guide panel disposed along an outer periphery of the worn wheel. The guide panel facilitates moving the belt along a rotation track of the worm wheel and feeding the belt into the space.

The self-propellable apparatus may further include a belt rewinding mechanism for rewinding the belt drawn out from the feeding roller onto the feeding roller, by rotating the feeding roller in a direction opposite to a direction of drawing out the belt.

The belt is preferably made of biocompatible plastic. The belt drawing-out mechanism is preferably remote controlled.

The self-propellable apparatus of the present invention has simple structure using the belts with the ends, and results in reduction of manufacturing cost. The inflation of the balloon and pressing the belts against the interior wall of the body lumen allow the production of the enough propulsive force to the insert section. Furthermore, when the insert section is moved by manipulation through an easy section without the necessity of the propulsive force, the balloon is deflated to reduce a physical burden on a patient.

### BRIEF DESCRIPTION OF THE DRAWINGS

For more complete understanding of embodiments of the present invention, and the advantage thereof, reference is now made to the following descriptions taken in conjunction with the accompanying drawings, in which:
Fig. 1 is a schematic view showing an electronic endoscope and a self-propellable apparatus;
Fig. 2 is a perspective view of a front end portion of the electronic endoscope and a propelling unit;
Fig. 3 is an exploded perspective view of the propelling unit;
Fig. 4 is a sectional view of the propelling unit in a state where a balloon is deflated and belts are wound on feeding rollers;
Fig. 5 is a sectional view of the propelling unit in a state before the start of self-propelling operation in which the balloon is inflated and the belts are wound on the feeding rollers;
Fig. 6 is a sectional view of the propelling unit in a state after the completion of the self-propelling operation in which the balloon is inflated and the belts are wound on winding rollers; and
Fig. 7 is a sectional view of the propelling unit according to another embodiment.

### DESCRIPTION OF THE PREFERRED EMBODIMENTS

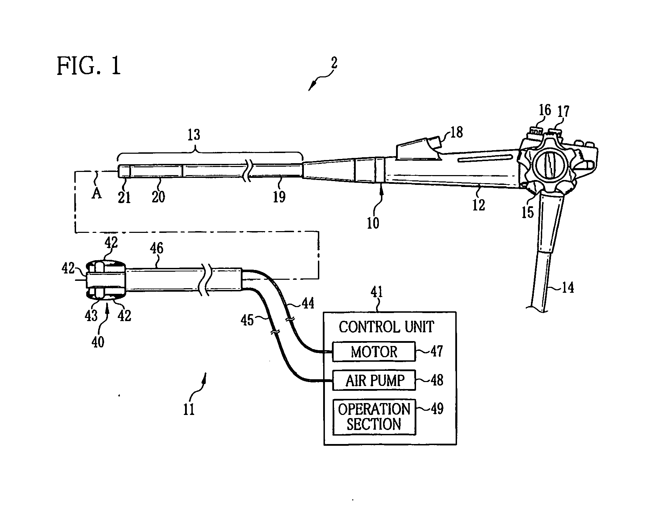

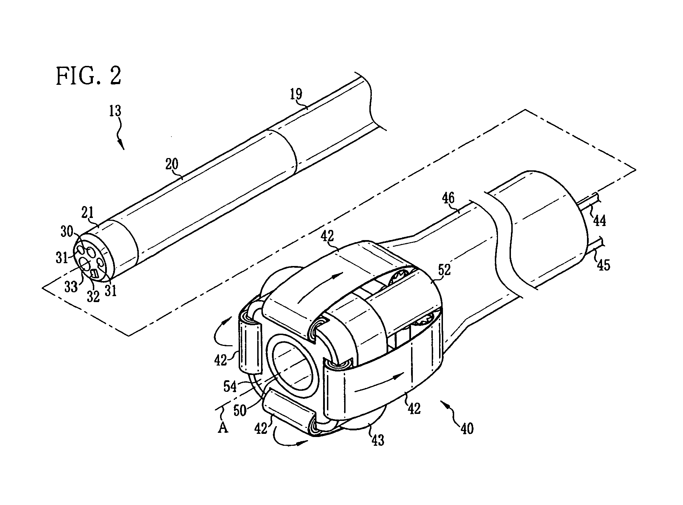

An endoscope system 2 is constituted of an electronic endoscope 10, a light source device (not shown), and a processor device (not shown). As shown in Figs. 1 and 2, a self-propellable apparatus 11 is attached to the electronic endoscope 10. The electronic endoscope 10 is provided with a handling section 12 and an insert section 13, which is coupled to the handling section 12 and introduced into a body lumen (for example, large intestine). To the handling section 12, a universal cord 14 is connected. The universal cord 14 is connected to the light source device and the processor device.

The handling section 12 is provided with an angle knob 15, an air/water supply button 16 for ejecting air and water from a front end of the insert section 13, a suction button 17, and the like. The handling section 12 has a medical instrument inlet 18 on the side of the insert section 13. Into the medical instrument inlet 18, a medical instrument such as forceps or an electric cautery is inserted.

The insert section 13 includes a flexible soft portion 19, a flexible bending portion 20, and a front end rigid portion 21 in this order from the side of the handling section 12 to a forward direction. The soft portion 19 has a length of several meters so as to make the front end rigid portion 21 reach a location of interest in the body lumen. The bending portion 20 is flexibly bent upward and downward and from side to side in response to operation of the angle knob 15 on the handling portion 12. Thereby, the front end rigid portion 21 is aimed at a desired direction inside a patient's body.

In the front end rigid portion 21, an imaging window 30 is formed to take an image of an interior of the body lumen therethrough. An objective optical system and a solid-state image sensor such as a CCD or CMOS image sensor, which captures the image of the interior of the body lumen, are disposed behind the imaging window 30. The solid-state image sensor is connected to the processor device through a signal cable that is routed through the insert section 13, the handling section 12, and the universal cord 14. The image of the interior of the body lumen is formed on a light receiving plane of the solid-state image sensor, and converted into an image signal in the solid-state image sensor. The processor device applies various types of image processing to the image signal received from the solid-state image sensor through the signal cable, and converts the image signal into video signal. The video signal is displayed as an observation image on a monitor (not shown) connected through a cable.

The front end rigid portion 21 is also provided with lighting windows 31, an air/water supply nozzle 32, and a medical instrument outlet 33. Illumination light is applied from the light source device through the lighting windows 31 to the interior of the body lumen. The air/water supply nozzle 32 ejects air and water from an air/water supplier contained in the light source device to the imaging window 30 in response to the operation of the air/water supply button 16. From the medical instrument outlet 33, a distal end of the medical instrument inserted into the medical instrument inlet 18 is exposed.

The self-propellable apparatus 11 is attached to the front end portion of the insert section 13 to help forward and backward movement of the insert section 13 inside the body lumen. The self-propellable apparatus 11 is constituted of a propelling unit 40 attached to the insert section 13 and a control unit 41 for controlling actuation of the propelling unit 40. The propelling unit 40 is provided with belts 42 and a balloon 43. The self-propellable apparatus 11 inflates the balloon 43 so as to press the belts 42 against the interior wall of the large intestine, for example, and moves the belts 42 backward i.e. in a direction opposite to an insertion direction with keeping the balloon in an inflation state to propel the insert section 13 into the depths of the intestine.

To the propelling unit 40, a torque wire 44 for supplying driving force to the belts 42 and an air pipe 45 for inflating and deflating the balloon 43 are connected. A sheath 46 extendable in the direction (insertion direction) of a central axis A of the insert section 13 is coupled to a rear end of the propelling unit 40. The sheath 46 integrates the insert section 13, the torque wire 44, and the air pipe 45. The insert section 13, the torque wire 44, and the air pipe 45 extend within the sheath 46 together. Note that, the sheath 46 may be separated from the propelling unit 40.

The control unit 41 has a motor 47, an air pump 48, and an operation section 49. The motor 47 is connected to the torque wire 44, and rotates the torque wire 44 to drive the belts 42. The air pump 48 is connected to the air pipe 45. The air pump 48 feeds air into the balloon 43, and exhausts air from the balloon 43 through the air pipe 45.

The operation section 49 controls the motor 47 and the air pump 48, in order to command the movement and stop of the propelling unit 40 and the start and stop of inflation or deflation of the balloon 43. Thereby, the propelling unit 40 is remote controlled by the control unit 41 disposed outside the patient's body.

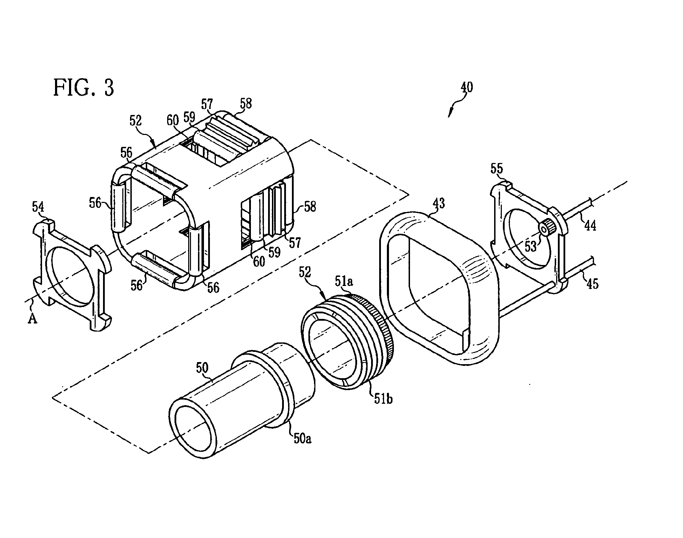

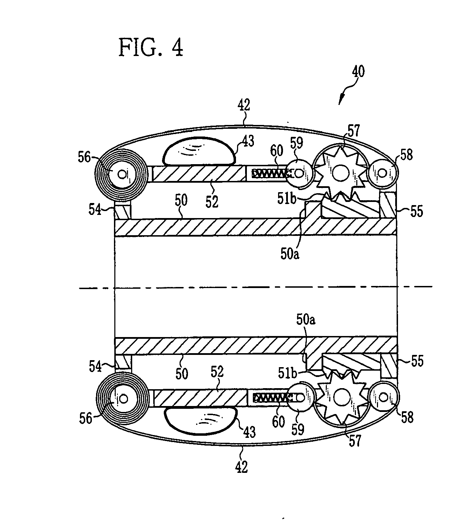

Next, the concrete structure of the propelling unit 40 will be described with referring to Figs. 3 and 4. Note that, Fig. 3 omits illustrating the sheath 46 and the belts 42. Fig. 4 omits illustrating the sheath 46. The propelling unit 40 includes a fixed cylinder 50, a gear barrel 51, and a belt holder 52. The fixed cylinder 50 has a cylindrical shape the inside diameter of which is approximately equal to the outside diameter of the insert section 13, and is securely fitted on the outer periphery of the insert section 13. The gear barrel 51 is formed into a cylindrical shape the inside diameter of which is slightly larger than the outside diameter of the fixed cylinder 50. The gear barrel 51 is rotatably attached and held on the outer periphery of the fixed cylinder 50. The gear barrel 51 includes a spur gear 51a and a worm gear 51b. The spur gear 51a is engaged with a gear 53 attached to a front end of the torque wire 44, so that the gear barrel 51 rotates in conjunction with the rotation of the gear 53 around the outer periphery of the fixed cylinder 50.

The belt holder 52 having the shape of an approximately square tube is disposed outside the fixed cylinder 50 and the gear barrel 51 so as to enclose them. The belt holder 52 is secured to the fixed cylinder 50 with front and back retaining plates 54 and 55, which join the inner periphery of the belt holder 52 to the outer periphery of the fixed cylinder 50. Note that, the gear barrel 51 is situated between a flange 50a of the fixed cylinder 50 and the back retaining plate 55.

The belt holder 52 is provided with four feeding rollers 56. On each feeding roller 56, the belt 42 is wound. The belt 42 is made of biocompatible plastic with flexibility, such as polyvinyl chloride, polyamide resin, fluorocarbon polymers, or polyurethane resin. The belt 42 is not an endless belt. One end of the belt 42 is secured to the feeding roller 56, and the other end of the belt 42 is secured to a winding roller 59, as described later on. Note that, in this embodiment, the belt 42 has a length enough to move the insert section 13 through the sigmoid colon of the large intestine, for example, a length of several tens of centimeters.

The feeding rollers 56 are disposed at the front of the belt holder 52 so as to be rotatable about axes orthogonal to the insertion direction. Each feeding roller 56 is biased by a not-shown spring (for example, spiral spring) in a direction of winding up the belt 42 (counterclockwise direction in Fig. 4). The bias prevents the belts 42 from being drawn out from the feeding rollers 56, when worm wheels 57 are not rotated, as details will be described later on.

The belt holder 52 is provided with belt drawing-out mechanisms each for drawing out the belt 42 from the feeding roller 56. Each belt drawing-out mechanism includes the worm wheel 57, a driven roller 58, and the winding roller 59. The belt holder 52 includes four sets of the worm wheels 57, the driven rollers 58, and the winding rollers 59, one set for each feeding roller 56. The worm wheel 57, the driven roller 58, and the winding roller 59 are disposed rotatably about axes parallel to the axis of the corresponding feeding roller 56.

The worm wheels 57 are engaged with the worm gear 51b, and rotate in conjunction with the rotation of the worm gear 51b. The driven roller 58 is disposed behind the worm wheel 57. The gap between the driven roller 58 and the worm wheel 57 is slightly narrower than the thickness of the belt 42. The winding roller 59 is disposed in front of the worm wheel 57. The winding roller 59 is held slidably in the insertion direction, and is biased by a spring 60 toward the worm wheel 57. Thus, the driven roller 58 and the winding roller 59 press the belt 42 against the worm wheel 57.

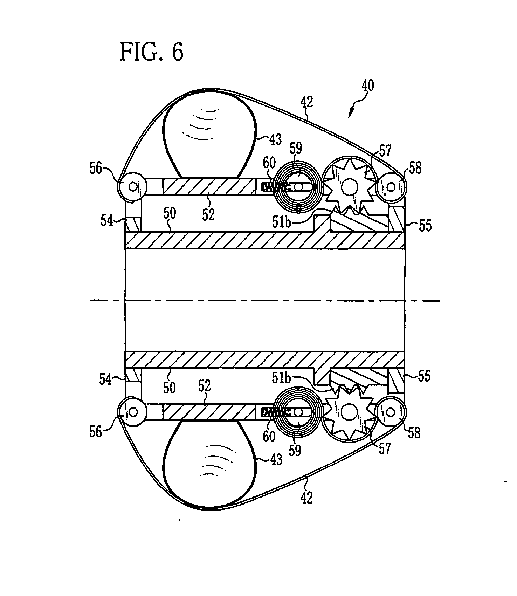

The belt 42 drawn out from the feeding roller 56 is looped over the driven roller 58 from behind (from the far side of the insert section 13). Then, the belt 42 passes through the gap between the driven roller 58 and the worm wheel 57, and reaches the winding roller 59 along the worm wheel 57. The belt 42 passes through the gap between the worm wheel 57 and the winding roller 59, and is secured to the winding roller 59 at its end. When the worm wheel 57 rotates in the counterclockwise direction in Fig. 4, the driven roller 58 and the winding roller 59 rotate in the clockwise direction in Fig. 4. Thereby, the belt 42 is drawn out from the feeding roller 56, and wound up onto the winding roller 59 (see Fig. 6). Note that, the teeth of the worm wheel 57 are exaggerated in height in the drawing, but are actually low in height and large in number for the purpose of preventing a scratch on the belt 42.

On the outer periphery of the belt holder 52, a balloon 43 is provided. The balloon 43, being a doughnut-shaped bladder, is fixed on the outer periphery of the belt holder 52 so as to be positioned under (on the side of the insert section 13) the belts 42 drawn out from the feeding rollers 56. The air pipe 45 penetrates the back retaining plate 55 through an opening, and extends to the inside of the belt holder 52, and is connected to the balloon 43 through an opening formed in the belt holder 52.

The operation of the endoscope system 2 having the above structure will be described. First, the insert section 13 is fitted into the fixed cylinder 50, so the propelling unit 40 is secured to the insert section 13. Then, the processor device, the light source device, and the like are turned on. Information as to the patient, examination, and the like is inputted. After that, the insert section 13 of the electronic endoscope 10 is introduced into a natural orifice of the body lumen e.g. the large intestine of the patient.

When the insert section 13 is introduced by the manipulation just before the sigmoid colon, for example, into which the insert section 13 is difficult to insert by the manipulation, the control unit 41 is turned on. Then, a forward movement command is inputted from the operation section 49. In response to the forward movement command, the motor 47 is actuated. The actuation of the motor 47 rotates the gear barrel 51 in the counterclockwise direction in Fig. 4 through the torque wire 44, the gear 53, and the spur gear 51a. The rotation of the gear barrel 51 causes the rotation of the worm wheel 57 through the worm gear 51b. Since the worm wheel 57 rotates the driven roller 58 and the winding roller 59 through the belt 42, the belt 42 is drawn out from the feeding roller 56 and wound onto the winding roller 59. By the movement of the belts 42, propulsive force occurs in the insert section 13, so the insert section 13 moves forward along the interior wall of the sigmoid colon.

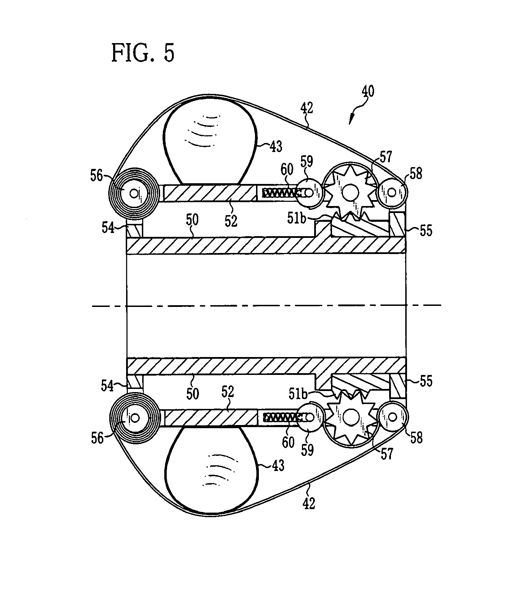

In a case where the friction between the belts 42 and the interior wall of the sigmoid colon is too small to produce the enough propulsive force to the insert section only through the actuation of the belts 49 by the motor 47, a balloon inflation command is inputted from the operation section 49. In response to the balloon inflation command, the air pump 48 is actuated to inflate the balloon 43, as shown in Fig. 5.

When the balloon 43 is inflated to the extent of producing the enough propulsive force through the belts 42, an inflation stop command is inputted from the operation section 49. Thus, the balloon 43 is kept in an inflated state. When the forward movement command is inputted in this state, the belts 42 are wound up onto the winding rollers 59 while being pressed against the interior wall of the sigmoid colon by the inflated balloon 43. Thus, it is possible to prevent idling of the belts 42, and reliably obtain the propulsive force.

When the front end rigid portion 21 has arrived at the location of interest or has passed through a section into which the insert section 13 is difficult to insert by the manipulation, a movement stop command is inputted from the operation section 49. Thus, the motor 47 stops rotating, and the production of the propulsive force to the insert section 13 is stopped. When a balloon deflation command is inputted from the operation section 49, the air is exhausted from the balloon 43 to deflate the balloon 43.

According to the present invention, as described above, the self-propellable apparatus has the simple structure using the belts with ends, and results in cost reduction. The inflation of the balloon allows the production of the enough propulsive force to the insert section. Furthermore, in a case where the insert section is easily moved forward or backward by the manipulation, the balloon is deflated to smoothly move the insert section and reduce a physical burden on the patient.

Note that, the detailed structure of the present invention is not limited to the above embodiment but appropriately changeable as long as the propulsive force is produced to the insert section through the belts having the ends. For example, the self-propellable apparatus may be provided with a sensor for detecting the volume of air flowing into the balloon or the internal pressure of the balloon, and the size of inflation of the balloon may be adjusted based on data from the sensor.

The balloon may be automatically inflated into an optimal size only by inputting a balloon use command. In this case, in response to the input of the balloon use command, the control unit may carry out a process for monitoring the volume of flow or the internal pressure of the balloon while air flows into the balloon, a process for judging whether or not the size of the balloon is optimal based on variation in the volume of flow or the internal pressure, and a process for stopping the air flow when the size of the balloon is judged to be optimal.

Instead of separately controlling the belts (motor) and the balloon (air pump), for example, a process of actuating the belts after the balloon is inflated to the predetermined size may be automatically carried out in response to the input of the forward movement command. Furthermore, a program may be created in advance in accordance with the location of interest or the like, and the motor and the air pump may be automatically controlled according to the program.

In the above embodiment, the four sets of the feeding rollers and the belt drawing-out mechanisms are provided, and the electronic endoscope is propelled with the four belts. However, for example, three or less sets of the feeding rollers and the belt drawing-out mechanisms may be provided, and the electronic endoscope may be propelled with the three or less belts. As a matter of course, five or more sets of the feeding rollers and the belt drawing-out mechanisms may be provided, and the electronic endoscope may be propelled with the five or more belts.

In the above embodiment, all the belts are pressed against the interior wall of the body lumen with use of the single balloon. However, the balloon may be provided for each belt, and each balloon may press the corresponding belt against the interior wall of the body lumen. As a matter of course, the number of the air pumps may be increased in accordance with the number of the balloons, or a switching valve may be provided to switch between the balloons into which the air flows, such that the size of inflation is adjustable separately from balloon to balloon.

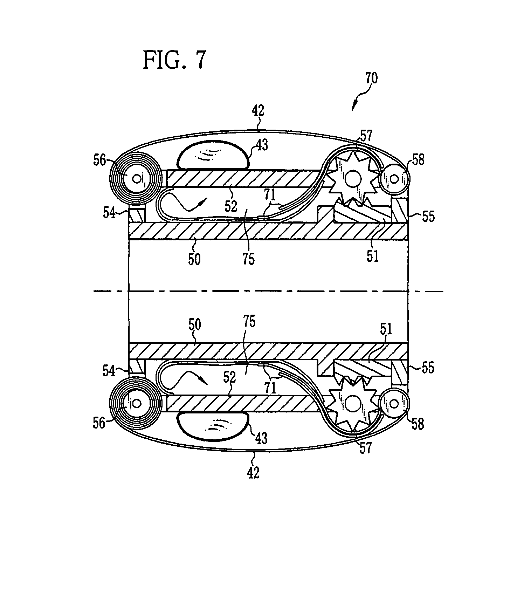

In the above embodiment, the belts drawn out from the feeding rollers are wound up onto the winding rollers. However, as shown in Fig. 7, the belts 42 drawn out from the feeding rollers 56 may be simply guided to space 75 provided inside the propelling unit 70. In this embodiment, the belts 42 are guided to the space 75 behind the feeding rollers 56, and guide panels 71 are provided to regulate the motion of the guided belts 42. In Fig. 7, the same reference numerals as those of the above embodiment refer to components identical or similar to those of the above embodiment, and the description thereof is omitted.

In the above embodiment, the feeding roller is biased by the spring in the direction of winding up the belt in order to prevent the belt from being drawn out from the feeding roller without the rotation of the worm wheel. However, the rotation of the feeding roller may be regulated by a stopper, for example. In this case, the stopper may be shiftable between a blocking position for blocking the rotation of the feeding roller and a release position for allowing the rotation of the feeding roller. The stopper may be operated from outside the patient's body with a wire. When the insert section arrives at the section in which the insertion by the manipulation is difficult, the wire is operated to shift the stopper to the release position.

Furthermore, in the above embodiment, the belts are actuated only in the section where the insertion by the manipulation is difficult. However, the belts may be actuated from the orifice to propel the insert section into the body lumen. However, in a case where the belts are actuated from the orifice, where the difficult-to-insert section is long, where there are plural difficult-to-insert sections to be passed through, or the like, the entire length of the belts can be possibly drawn out before the insert section arrives at the location of interest, and propulsive force may become unusable in midcourse. To solve this problem, belt rewinding mechanisms that rewind up the belts drawn out from the feeding rollers again onto the feeding rollers may be provided in order to allow the repeated production of the propulsive force.

In this case, each belt rewinding mechanism may include the spring for biasing the feeding roller in the direction of winding up the belt, as described above. While the balloon is deflated, the motor is rotated. Thereby, the belts are rewound onto the feeding rollers, and the propulsive force can be supplied again. In another case, another type of the belt rewinding mechanisms that are similar to the belt drawing-out mechanisms of the above embodiment, in other words, each having the motor, the torque wire, the worm gear, and the like may be provided so as to move and rewind the belts in the opposite direction.

The self-propellable apparatus is attached to the electronic endoscope for medical use in the above embodiment, but is available with any instrument for imaging the interior of a conduit, a duct, or the like such as an electronic endoscope for industrial use or an ultrasonic probe.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention as defined in independent claim 1, they should be construed as included therein.

## Claims

1. A self-propellable apparatus (11) attached to a front end portion of an insert section (13) of an electronic endoscope (10), for propelling said front end portion in a body lumen by power from an outside power source, said self-propellable apparatus (11) comprising:
a belt, **characterized by**:
a feeding roller (56) on which one end of the belt (42) is wound; and
a belt drawing-out mechanism (57) disposed behind said feeding roller (56), adapted to draw out said belt (42) from said feeding roller (56) to produce a propulsive force to said front end portion, when said belt (42) is pressed against an interior wall of the body lumen.

2. The self-propellable apparatus (11) according to claim 1, further comprising:
a balloon (43) disposed on an inner side of said belt (42), adapted to press said belt (42) against an interior wall of said body lumen, said balloon (43) being changeable between an inflated state and a deflated state.

3. The self-propellable apparatus (11) according to claim 1, wherein a plurality of sets each of which includes said feeding roller (56) and said belt drawing-out mechanism (57) are provided around a periphery of said insert section (13).

4. The self-propellable apparatus (11) according to claim 2, wherein a plurality of sets each of which includes said feeding roller (56), said belt drawing-out mechanism (57), and said balloon (43) are provided around a periphery of said insert section (13).

5. The self-propellable apparatus (11) according to claim 1, wherein said belt drawing-out mechanism includes a worm wheel (57) rotated by said outside power source to move said belt (42).

6. The self-propellable apparatus (11) according to claim 5, wherein said belt drawing-out mechanism further includes:
a driven roller (58) disposed behind said worm wheel (57), for turning said belt (42) drawn out from said feeding roller (56) to said worm wheel (57) and pressing said belt (42) against said worm wheel (57).

7. The self-propellable apparatus (11) according to claim 6, further comprising:
a fixed cylinder (50) fitted onto said front end portion of said insert section (13);
a gear barrel (51) rotatably fitted on said fixed cylinder (50), said worm wheel (57) being disposed on an outer periphery of said gear barrel (51); and
a belt holder (52) held by said fixed cylinder (50), said belt holder (52) having said feeding roller (56), said worm wheel (57), and said driven roller (58) attached thereto.

8. The self-propellable apparatus (11) according to claim 7, wherein said belt drawing-out mechanism further includes:
a winding roller (59) to which the other end of said belt (42) is secured, said winding roller (59) being disposed in an opposite side of said driven roller (58) with respect to said worm wheel (57), and rotated in conjunction with said worm wheel (57) to wind up said belt (42) fed from said worm wheel (57).

9. The self-propellable apparatus (11) according to claim 8, wherein said winding roller (59) steps back against a bias of a spring (60) by a distance corresponding to a winding diameter of said belt (42).

10. The self-propellable apparatus (11) according to claim 7, wherein said belt drawing-out mechanism further includes:
space (75) formed between said fixed cylinder (50) and said belt holder (52), for holding said belt (42) fed from said worm wheel (57).

11. The self-propellable apparatus (11) according to claim 10, wherein said belt drawing-out mechanism further includes:
a guide panel (71) disposed along an outer periphery of said worn wheel (57), for facilitating moving said belt (42) along a rotation track of said worm wheel (57) and feeding said belt (42) into said space (75).

12. The self-propellable apparatus (11) according to claim 1, further comprising:
a belt rewinding mechanism for rewinding said belt (42) drawn out from said feeding roller (56) onto said feeding roller (56), by rotating said feeding roller (56) in a direction opposite to a direction of drawing out said belt (42).

13. The self-propellable apparatus (11) according to claim 1, wherein said belt (42) is made of biocompatible plastic.

14. The self-propellable apparatus (11) according to claim 1, wherein said belt drawing-out mechanism (57) is remote controlled.

## Patentansprüche

1. Selbstvortreibende Vorrichtung (11), die an einem vorderen Endbereich eines Einführabschnitts (13) eines elektronischen Endoskops (10) befestigt ist, um den vorderen Endbereich in einem Körperhohlraum mittels Energie aus einer äußeren Energiequelle vorzutreiben, wobei die selbstvortreibende Vorrichtung (11) umfasst:
einen Riemen,
**gekennzeichnet durch**:
eine Abgaberolle (56), auf der ein Ende des Riemens (42) aufgewickelt ist; und
einen Riemenabziehmechanismus (57), der hinter der Abgaberolle (56) angeordnet und dazu ausgebildet ist, den Riemen (42) von der Abgaberolle (56) abzuziehen, um eine Vortriebskraft für den vorderen Endbereich zu erzeugen, wenn der Riemen (42) gegen eine Innenwand des Körperhohlraums gedrückt wird.

2. Selbstvortreibende Vorrichtung (11) nach Anspruch 1, weiterhin umfassend:
einen an einer Innenseite des Riemens (42) angeordneten Ballon (43), ausgebildet zum Anpressen des Riemens (42) gegen eine Innenwand des Körperhohlraums, wobei der Ballon (43) zwischen einem aufgepumpten Zustand und einem entleerten Zustand wechseln kann.

3. Selbstvortreibende Vorrichtung (11) nach Anspruch 1, bei der eine Mehrzahl von Sätzen mit jeweils der Abgaberolle (56) und dem Riemenabziehmechanismus (57) um einen Umfang des Einführabschnitts (13) herum vorgesehen sind.

4. Selbstvortreibende Vorrichtung (11) nach Anspruch 2, bei der eine Mehrzahl von Sätzen mit jeweils der Abgaberolle (56), dem Riemenabziehmechanismus (57) und dem Ballon (43) um einen Umfang des Einführabschnitts (13) herum angeordnet ist.

5. Selbstvortreibende Vorrichtung (11) nach Anspruch 1, bei der der Riemenabziehmechanismus ein von der äußeren Energiequelle gedrehtes Schneckenrad (57) zum Bewegen des Riemens (42) enthält.

6. Selbstvortreibende Vorrichtung (11) nach Anspruch 5, bei der der Riemenabziehmechanismus weiterhin enthält:
eine angetriebene Rolle (58), die hinter dem Schneckenrad (57) angeordnet ist, um den von der Abgaberolle (56) abgezogenen Riemen (42) zu dem Schneckenrad (57) zu wenden und den Riemen (42) gegen das Schneckenrad (57) anzudrücken.

7. Selbstvortreibende Vorrichtung (11) nach Anspruch 6, weiterhin umfassend:
einen an den vorderen Endbereich des Einführabschnitts (13) angepassten festen Zylinder (50);
eine drehbar an dem festen Zylinder (50) gelagerte Getriebehülse (51), wobei das Schneckenrad (57) an einem Außenumfang der Getriebehülse (51) angeordnet ist; und
einen Riemenhalter (52), der von dem festen Zylinder (50) gehalten wird, wobei an dem Riemenhalter (52) die Abgaberolle (56), das Schneckenrad (57) und die angetriebene Rolle (58) angebracht sind.

8. Selbstvortreibende Vorrichtung (11) nach Anspruch 7, bei der der Riemenabziehmechanismus weiterhin enthält:
eine Wickelrolle (59), an der das andere Ende des Riemens (42) befestigt ist, wobei die Wickelrolle (59) auf der entgegengesetzten Seite der angetriebenen Rolle (58) bezüglich des Schneckenrads (57) angeordnet ist und im Verein mit dem Schneckenrad (57) dreht, um den von dem Schneckenrad (57) zugeführten Riemen (42) aufzuwickeln.

9. Selbstvortreibende Vorrichtung (11) nach Anspruch 8, bei der die Wickelrolle (59) gegen die Vorspannkraft einer Feder (60) um eine Strecke zurückweicht, die einem Wickeldurchmesser des Riemens (42) entspricht.

10. Selbstvortreibende Vorrichtung (11) nach Anspruch 7, bei der der Riemenabziehmechanismus weiterhin enthält:
einen Raum (75), der zwischen dem festen Zylinder (50) und dem Riemenhalter (52) ausgebildet ist, um den von dem Schneckenrad (57) zugeführten Riemen (42) zu halten.

11. Selbstvortreibende Vorrichtung (11) nach Anspruch 10, bei der der Riemenabziehmechanismus weiterhin enthält:
eine Führungsplatte (71), die entlang einem Außenumfang des Schneckenrads (57) angeordnet ist, um die Bewegung des Riemens (42) entlang einer Drehbahn des Schneckenrads (57) zu erleichtern und den Riemen (42) in den Raum (75) zu leiten.

12. Selbstvortreibende Vorrichtung (11) nach Anspruch 1, weiterhin umfassend:
einen Riemenrückwickelmechanismus zum Zurückwickeln des von der Abgaberolle (56) abgezogenen Riemens (42) auf die Abgaberolle (56), indem die Abgaberolle (56) in eine zu der Abziehrichtung des Riemens (42) entgegengesetzten Richtung gedreht wird.

13. Selbstvortreibende Vorrichtung (11) nach Anspruch 1, bei der der Riemen (42) aus biokompatiblem Kunststoff besteht.

14. Selbstvortreibende Vorrichtung (11) nach Anspruch 1, bei der der Riemenabziehmechanismus (57) ferngesteuert wird.

## Revendications

1. Appareil automoteur (11) fixé à une partie d'extrémité avant d'une section d'insertion (13) d'un endoscope électronique (10), pour faire avancer ladite partie d'extrémité avant dans une lumière corporelle avec une alimentation provenant d'une source d'alimentation extérieure, ledit appareil automoteur (11) comprenant :
une courroie, **caractérisée par** :
un rouleau d'alimentation (56) sur lequel une extrémité de la courroie (42) est enroulée ; et
un mécanisme de traction de courroie (57) disposé derrière ledit rouleau d'alimentation (56), adapté pour tirer ladite courroie (42) dudit rouleau d'alimentation (56) afin de produire une force de propulsion sur ladite partie d'extrémité avant, lorsque ladite courroie (42) est comprimée contre une paroi intérieure de la lumière corporelle.

2. Appareil automoteur (11) selon la revendication 1, comprenant en outre :
un ballonnet (43) disposé sur un côté interne de ladite courroie (42), adapté pour comprimer ladite courroie (42) contre une paroi intérieure de ladite lumière corporelle, ledit ballonnet (43) pouvant passer d'un état gonflé à un état dégonflé.

3. Appareil automoteur (11) selon la revendication 1, dans lequel une pluralité d'ensembles, dont chacun comprend ledit rouleau d'alimentation (56) et ledit mécanisme de traction de courroie (57), sont prévus autour d'une périphérie de ladite section d'insertion (13).

4. Appareil automoteur (11) selon la revendication 2, dans lequel une pluralité d'ensembles, dont chacun comprend ledit rouleau d'alimentation (56), ledit mécanisme de traction de courroie (57) et ledit ballonnet (43), sont prévus autour d'une périphérie de ladite section d'insertion (13).

5. Appareil automoteur (11) selon la revendication 1, dans lequel ledit mécanisme de traction de courroie comprend une roue à vis sans fin (57) entraînée en rotation par ladite source d'alimentation extérieure afin de déplacer ladite courroie (42).

6. Appareil automoteur (11) selon la revendication 5, dans lequel ledit mécanisme de traction de courroie comprend en outre :
un rouleau entraîné (58) disposé derrière ladite roue à vis sans fin (57) pour faire tourner ladite courroie (42) tirée dudit rouleau d'alimentation (56) à ladite roue à vis sans fin (57) et comprimer ladite courroie (42) contre ladite roue à vis sans fin (57).

7. Appareil automoteur (11) selon la revendication 6, comprenant en outre :
un cylindre fixe (50) monté sur ladite partie d'extrémité avant de ladite section d'insertion (13) ;
un corps cylindrique d'engrenage (51) monté en rotation sur ledit cylindre fixe (50), ladite roue à vis sans fin (57) étant disposée sur une périphérie externe dudit corps cylindrique d'engrenage (51) ; et
un support de courroie (52) maintenu par ledit cylindre fixe (50), ledit support de courroie (52) ayant ledit rouleau d'alimentation (56), ladite roue à vis sans fin (57) et ledit rouleau entraîné (58) fixés à ce dernier.

8. Appareil automoteur (11) selon la revendication 7, dans lequel ledit mécanisme de traction de courroie comprenant en outre :
un rouleau d'enroulement (59) sur lequel l'autre extrémité de ladite courroie (42) est fixée, ledit rouleau d'enroulement (59) étant disposé sur un côté opposé dudit rouleau entraîné (58) par rapport à ladite roue à vis sans fin (57), et entraîné conjointement avec ladite roue à vis sans fin (57) pour enrouler ladite courroie (42) fournie par ladite roue à vis sans fin (57).

9. Appareil automoteur (11) selon la revendication 8, dans lequel ledit rouleau d'enroulement (59) recule contre une sollicitation d'un ressort (60) d'une distance correspondant à un diamètre d'enroulement de ladite courroie (42).

10. Appareil automoteur (11) selon la revendication 7, dans lequel ledit mécanisme de traction de courroie comprend en outre :
un espace (75) formé entre ledit cylindre fixe (50) et ledit support de courroie (52) pour maintenir ladite courroie (42) fournie par ladite roue à vis sans fin (57).

11. Appareil automoteur (11) selon la revendication 10, dans lequel ledit mécanisme de traction de courroie comprend en outre :
un panneau de guidage (71) disposé le long d'une périphérie externe de ladite roue à vis sans fin (57) pour faciliter le déplacement de ladite courroie (42) le long d'un chemin de rotation de ladite roue à vis sans fin (57) et l'amenée de ladite courroie (42) dans ledit espace (75).

12. Appareil automoteur (11) selon la revendication 1, comprenant en outre :
un mécanisme de rembobinage de courroie pour rembobiner ladite courroie (42) tirée à partir dudit rouleau d'alimentation (56) sur ledit rouleau d'alimentation (56), en faisant tourner ledit rouleau d'alimentation (56) dans une direction opposée à une direction de traction de ladite courroie (42).

13. Appareil automoteur (11) selon la revendication 1, dans lequel ladite courroie (42) est réalisée avec un plastique biocompatible.

14. Appareil automoteur (11) selon la revendication 1, dans lequel ledit mécanisme de traction de courroie (57) est commandé à distance.
